# EUROPÄISCHE PATENTANMELDUNG

(11) **EP 2 596 769 A1**
(43) Veröffentlichungstag der Anmeldung: **29.05.2013**
(21) Anmeldenummer: 12190800.8
(22) Anmeldetag: 31.10.2012
(51) Int. Cl.: A61F 2/966

(54) **Freigabevorrichtung zum Lösen eines medizinisches Implantats von einen Katheter sowie Katheter mit einer Freigabevorrichtung**

(30) Priorität: 24.11.2011 US 201161563554 P
(71) Anmelder: Biotronik AG, 8180 Bülach (CH)
(72) Erfinder: Fargahi, Amir, 8180 Bülach (CH)
(74) Vertreter: Lindner-Vogt, Karin L.

(57) **Zusammenfassung**

Die Erfindung betrifft eine Freigabevorrichtung (100, 100a) zum Lösen eines medizinisches Implantats (105) von einer Einführvorrichtung (110), bei welcher das Implantat (105) durch eine Relativbewegung zwischen einem ersten und einem zweiten Einführelement (52, 54) freisetzbar ist, umfassend einen Körper (10) mit einem proximalen Ende (12), das im Benutzungszustand einem Anwender zugewandt ist und einem distalen Ende (14), das im Benutzungszustand vom Anwender entfernt ist, wobei zwischen dem proximalen und dem distalen Ende (12, 14) ein Aktor (16) vorgesehen ist, wobei der Aktor (16) zur Erzeugung einer gezielten Relativbewegung zwischen dem ersten und dem zweiten Einführelement (52, 54) der Einführvorrichtung (110) hydraulisch antreibbar ist. Die Erfindung betrifft ferner eine Einführvorrichtung (110) mit einer solchen Freigabevorrichtung (100, 100a).

## Beschreibung

Die Erfindung betrifft eine Freigabevorrichtung zum Lösen eines medizinischen Implantats von einem Katheter sowie einen Katheter mit einer Freigabevorrichtung zum Freigeben eines medizinischen Implantats zur Implantation in einen tierischen und/oder menschlichen Körper nach den Oberbegriffen der unabhängigen Patentansprüche.

In der Medizin kommen häufig Implantate zum Einsatz, die zur Erfüllung von Ersatzfunktionen permanent oder zumindest für einen längeren Zeitraum in einen tierischen und/oder menschlichen Körper eingebracht werden. Zu nennen wären hier beispielsweise Herzschrittmacher, Hirnschrittmacher für Parkinsonpatienten, Herzimplantate, Cochleaimplantate, Retina Implantate, zahnmedizinische Implantate, Implantate zum Gelenkersatz, Gefäßprotesen oder Stents.

Implantate sind zum Einführen in den Körper mit Kathetern verbunden und müssen am Einsatzort genau platziert und definiert freigegeben werden können. Hierzu ist beispielsweise bekannt, das Implantat durch eine Schiebbewegung freizugeben.

Der Erfindung liegt die Aufgabe zugrunde, eine Freigabevorrichtung anzugeben, mit der ein hochpräzises und gezieltes Freigeben eines Implantats erfolgen kann.

Eine weitere Aufgabe ist in der Bereitstellung einer entsprechenden Einführvorrichtung zu sehen.

Ferner besteht eine weitere Aufgabe darin, ein Verfahren zum Herstellen einer solchen Freigabevorrichtung bereit zu stellen.

Zudem ergibt sich eine weitere Aufgabe in der Bereitstellung eines Verfahrens zum Betreiben einer entsprechenden Freigabevorrichtung.

Die Aufgabe wird erfindungsgemäß durch die Merkmale der unabhängigen Ansprüche gelöst. Günstige Ausgestaltungen und Vorteile der Erfindung ergeben sich aus den weiteren Ansprüchen und der Beschreibung.

Es wird eine Freigabevorrichtung zum Lösen eines medizinischen Implantats von einer Einführvorrichtung vorgeschlagen, bei welcher das Implantat durch eine Relativbewegung zwischen einem ersten und einem zweiten Einführelement freisetzbar ist. Die Freigabevorrichtung umfasst einen Körper mit einem proximalen Ende, das im Benutzungszustand einem Anwender zugewandt ist und einem distalen Ende, das im Benutzungszustand vom Anwender entfernt ist, wobei zwischen dem proximalen und dem distalen Ende ein Aktor vorgesehen ist, und wobei der Aktor zur Erzeugung einer gezielten Relativbewegung zwischen dem ersten und dem zweiten Einführelement der Einführvorrichtung hydraulisch antreibbar ist.

Durch die erfindungsgemäße Ausgestaltung kann eine Freigabevorrichtung bereitgestellt werden, bei der große Kräfte einfach erzeugt werden können. Dadurch kann eine vom Anwender und/oder Arzt zu erbringende Kraft bedienerfreundlich minimiert werden. Der Anwender und/oder Arzt kann sich auf eine korrekte Positionierung des Implantats konzentrieren. Die Freigabe des Implantats wird dadurch präziser und schneller. All dies resultiert in einer hohen Erfolgsrate der Implantation. In diesem Zusammenhang soll unter "vorgesehen" speziell ausgestattet und/oder ausgelegt verstanden werden.

Des Weiteren wird vorgeschlagen, dass der Körper zumindest am distalen Ende ein Ventil aufweist. Das Ventil ist bevorzugt ein von außen betreibbares Ventil. Es kann jedoch zusätzlich ein in einer Richtung betreibbares mediengesteuertes Ventil sein, beispielsweise durch für eine Entlüftung, die aufgrund einer Druckerhöhung stattfindet. Im konstruktiv einfachsten Fall ist das Ventil als ein verschließbares Luer Lock ausgebildet. So können vorteilhaft genormten Bauteile und/oder Anschlussmaße zur Anwendung kommen. Vorteilhafterweise ist am proximalen Ende und am distalen Ende je ein Ventil vorgesehen, wodurch die Freigabevorrichtung Bauteil sparend für mehrere Betriebsmodi verwendet werden kann.

Ferner ist es vorteilhaft, wenn über das Ventil eine Hydraulikflüssigkeit zum Bewegen des Aktors in den Körper einbringbar ist. Durch diese Realisierung kann die Kraftaufbringung leicht reguliert und gesteuert werden. Wegen der geringen Kompressibilität der Hydraulikflüssigkeit kann eine besonders stabile Bewegung der Einführelement realisiert werden. Die Hydraulikflüssigkeit ist bevorzugt mittels einer Spritze einbringbar, wodurch ein konventionelles Mittel eingesetzt werden kann, dessen Funktionsweise bekannt ist. Somit entfällt eine zeitraubende Einarbeitungsphase. Die Hydraulikflüssigkeit ist bevorzugt Wasser. Durch das Wasser bzw. die Hydraulikflüssigkeit wird die Reibung im Körper vermindert, was die innere Reibung in der Freigabevorrichtung vorteilhaft minimiert.

In einer weiteren Ausgestaltung der Erfindung ist eine Geschwindigkeit der Relativbewegung zwischen dem ersten und dem zweiten Einführelement der Einführvorrichtung durch einen hydraulischen Druck vorgebbar. Hierdurch kann eine schnelle, gleichförmige und/oder stufenlos verstellbare bzw. kontrollierbare Geschwindigkeit realisiert werden. Grundsätzlich wäre auch eine Verstellung in Stufen denkbar. Zudem kann eine hohe Präzision bei der Positionierung des Implantats durch den Einsatz des Drucks erreicht werden, statt wie im Stand der Technik ein Schieben und Zurückziehen der Einführelemente einzusetzen. Ein auszubringender Druck ist hierbei abhängig von einer Länge und/oder einem Durchmesser des Körpers und/oder von den Abmessungen bzw. Wirkflächen des Aktors. Ein Fachmann wird diesen aufgrund seines Fachwissens selbständig auswählen.

Ferner wird vorgeschlagen, dass mindestens zwei Druckbereiche vorgesehen sind, wobei ein erster niederer Druckbereich für eine langsame Relativbewegung und ein zweiter, hoher Druckbereich für eine schnellere Relativbewegung vorgesehen ist. Dies erlaubt auf einfache Weise eine Umsetzung von zumindest zwei unterschiedlichen Geschwindigkeiten der Freigabevorrichtung. Zudem kann vorteilhaft ein einfacher Wechsel von schnellem auf langsamem Freigeben erfolgen und umgekehrt. Dies erlaubt eine unkomplizierte, einfache und schnelle Geschwindigkeitsregulierung bei der Freigabe des Implantats. Wird beispielsweise ein Druck von 2 bar an den Aktor angelegt, wird eine langsame Relativbewegung bzw. Freigabe vermittelt. Wird hingegen ein Druck von 6 bar angelegt, sind die Relativbewegung und damit die Freigabe schneller.

Des Weiteren kann es vorteilhaft sein, wenn der Aktor eine Durchführung für das innere Einführelement aufweist. Dies erlaubt eine kompakte Anordnung, welche das durchgeführte Einführelement stabilisiert und schützt. Ist die Einführvorrichtung ein Katheter, so kann das betreffende Einführelement ein Innenschaft des Katheters sein.

Eine effiziente Kraftübermittlung mit ausreichender Kontaktfläche kann vorteilhaft erreicht werden, wenn der Aktor eine Kolbenstange und einen Kolben aufweist. Zudem wird vorgeschlagen, dass der Kolben an einem proximalen Ende der Kolbenstange angeordnet ist, wodurch sich die Kolbenstange während der Bewegung Platz sparend in eine an den Körper distal anschließende Einführvorrichtung hineinbewegen kann. Somit ergibt sich eine vorteilhaft einfache und kompakte Konstruktion der Freigabevorrichtung.

Gemäß einem weiteren Aspekt der Erfindung wird vorgeschlagen, dass der Körper einen Hydraulikzylinder für den Kolben aufweist, wodurch sich eine stabile Konstruktion ergibt. Bevorzugt bildet der Körper den Hydraulikzylinder. Hierdurch können vorteilhaft Bauteile, Montageaufwand und Kosten eingespart werden. Zudem wird ein geringes Gewicht der Einführvorrichtung realisiert.

Eine bevorzugte Weiterbildung besteht darin, dass die Kolbenstange einstückig mit dem äußeren Einführelement, insbesondere bei einem Katheter dem Außenschaft, ausgeführt ist, wodurch die Relativbewegung besonders zuverlässig und exakt übermittelt werden kann. Ferner kann so Bauteile eingespart und das Gewicht weiter reduziert werden. Vorteilhafterweise ist der Kolben einstückig mit dem äußeren Einführelement ausgeführt. Dies erlaubt eine effiziente und direkte Kraftübertragung auf das Einführelement sowie eine Bauteil sparende und kompakte Bauweise der Einführvorrichtung.

Gemäß einer vorteilhaften Ausgestaltung weist die Freigabevorrichtung zumindest eine Dichtung auf, wodurch ein entweichen von Hydraulikflüssigkeit vorteilhaft und Prozess stabilisierend verhindert wird. Hierbei kann die Dichtung von jedem dem Fachmann für sinnvoll erachtetes Element gebildet sein, wie beispielsweise einem O-Ring. Die Dichtung ist bevorzugt aus einem Material gebildet, das gegenüber der Hydraulikflüssigkeit resistent ist und das gute Gleiteigenschaften, insbesondere auf einem Einführelement, aufweist. Des Weiteren wird vorgeschlagen, dass eine Dichtung zwischen zumindest einem Einführelement und dem Körper angeordnet ist. Hierdurch kann der Körper vorteilhaft gegenüber einer Umgebung des Körpers abgedichtet werden. Bevorzugt stellt das Einführelement im Falle eines Katheters den Außenschaft dar. Ferner ist es vorteilhaft, wenn eine Dichtung zwischen einem Einführelement und dem Aktor angeordnet ist, wodurch verhindert wird, dass Hydraulikflüssigkeit zwischen die Einführelemente gelangt. Das Einführelement stellt im Falle eines Katheters bevorzugt den Innenschaft dar. Gemäß einer vorteilhaften Ausgestaltung kann ein Lumen des inneren Einführelements ein Entlüftungsventil aufweisen.

In einer weiteren Ausgestaltung der Erfindung wird vorgeschlagen, dass der Körper als ein Gehäuse ausgeführt ist, wodurch der Körper und die Freigabevorrichtung sehr stabil ausgeführt sein können. Das Gehäuse kann insbesondere einen Handgriff der Freigabevorrichtung bzw. der Einführvorrichtung bilden. Dies ermöglicht eine leichte und kontrollierte Bedienbarkeit der Freigabevorrichtung. Das Gehäuse ist vorteilhaft in wenigstens einem Bereich durchsichtig ausgeführt, wodurch der Fortschritt der Bewegung des Aktors und damit des Einführelements besonders einfach überwacht werden kann. Bevorzugt ist das gesamte Gehäuse durchsichtig ausgeführt.

In einer bevorzugten Ausgestaltung ist die Geschwindigkeit der Relativbewegung optisch überwachbar. Dies kann besonders einfach durch die Überwachung der Bewegung des farbigen (z.B. schwarzen) Kolbens im durchsichtigen Gehäuse bzw. Zylinder erfolgen. Hierfür weist das Gehäuse bzw. der Zylinder bevorzugt einen Maßstab, wie beispielsweise eine mL-Skala, auf.

Ferner wird vorgeschlagen, dass eine Geschwindigkeit der Relativbewegung mittels eines Spritzenmanometers überwachbar und/oder regulierbar ist. Dies ermöglicht eine einfache Anzeige der Belastung. Ferner kann hierdurch ein weiteres Kontrollmittel zur Verfügung gestellt werden, das insbesondere unabhängig von einer Beobachtung der Aktorbewegung ist. Diese Beobachtung kann zudem von einer weiteren Person als dem Anwender der Freigabevorrichtung durchgeführt werden und ist unabhängig von einer behinderungsfreien Sicht auf den Körper bzw. das Gehäuse.

Es wird des Weiteren eine Einführvorrichtung zum Einführen eines medizinischen Implantats, welches durch eine Relativbewegung zwischen einem ersten und einem zweiten Einführelement freisetzbar ist, vorgeschlagen. Die Einführvorrichtung umfasst eine Freigabevorrichtung zum Lösen des medizinischen Implantats, die einen Körper mit einem proximalen Ende, das im Benutzungszustand einem Anwender zugewandt ist und einem distalen Ende, das im Benutzungszustand vom Anwender entfernt ist, aufweist, wobei zwischen dem proximalen und dem distalen Ende ein Aktor vorgesehen ist und der Aktor zur Erzeugung einer gezielten Relativbewegung zwischen dem ersten und dem zweiten Einführelement der Einführvorrichtung hydraulisch antreibbar ist.

Durch die erfindungsgemäße Ausgestaltung kann eine Einführvorrichtung bereitgestellt werden, bei der große Kräfte unabhängig von der vom Anwender bzw. Arzt zu erbringenden Kraft einfach erzeugt werden können. Zudem weist sie eine einfache Handhabung und ein optimiertes Design auf. Die Freigabe des Implantats wird dadurch präziser und schneller. All dies resultiert in einer hohen Erfolgsrate der Implantation. Die Einführvorrichtung kann günstigerweise ein Katheter sein. Besonders vorteilhaft kann die Einführvorrichtung zur Montage und Freigabe einer Prothese, einer Herzklappe oder eines Stents eingesetzt werden.

Es wird ferner ein Verfahren zum Herstellen einer Implantat aufweisenden Freigabevorrichtung vorgeschlagen. Die Freigabevorrichtung umfasst einen Körper mit einem proximalen Ende, das im Benutzungszustand einem Anwender zugewandt ist und einem distalen Ende, das im Benutzungszustand vom Anwender entfernt ist, wobei zwischen dem proximalen und dem distalen Ende ein Aktor vorgesehen ist. Ferner dient die Freigabevorrichtung zum Lösen eines medizinischen Implantats von einer Einführvorrichtung bei welcher das Implantat durch eine Relativbewegung zwischen einem ersten und einem zweiten Einführelement freisetzbar ist. Das Verfahren weist zumindest die folgenden Schritte auf: Platzieren des Aktors am distalen Ende des Körpers; Betätigen des Aktors durch Anlegen eines hydraulischen Drucks an einem distalen Ende des Körpers und damit Freilegen des Einführelements am distalen Ende der Einführvorrichtung durch Bewegen des Einführelements in Richtung eines proximalen Endes der Einführvorrichtung; Befestigen des Implantats am freigelegten Einführelement; Betätigen des Aktors durch Anlegen eines hydraulischen Drucks am proximalen Ende des Körpers und damit Bedecken des Implantats durch das in Richtung des distalen Endes der Einführvorrichtung verschobenen Einführelement durch dieses.

Durch die erfindungsgemäße Ausgestaltung kann ein Verfahren realisiert werden, mittels dem eine Freigabevorrichtung bedienerfreundlich, zuverlässig und schnell hergestellt werden kann. In diesem Zusammenhang umfasst der Begriff "Betätigen" auch den Sachverhalt des Verschiebens des Aktors. Im Falle des Freilegens des Einführelements - insbesondere des Innenschafts - am distalen Ende der Einführvorrichtung umfasst das Betätigen ein Verschieben des Aktors in Richtung des proximalen Endes des Körpers. Ferner umfasst im Falle des Bedeckens des Implantats durch das in Richtung des distalen Endes verschobenen Einführelement - insbesondere der Außenschaft - ein Betätigen ein Verschieben des Aktors in Richtung des distalen Endes des Körpers. Unter "Anlegen eines hydraulischen Drucks soll hier verstanden werden, dass ein Druck angelegt wird, der über Atmosphärendruck ist, beispielsweise bei 2 oder 6 bar. Dieses Druckanlegen kann stufenweise oder kontinuierlich erfolgen.

Zudem wird ein Verfahren zum Betreiben einer Freigabevorrichtung vorgeschlagen. Die Freigabevorrichtung umfasst einen Körper mit einem proximalen Ende, das im Benutzungszustand einem Anwender zugewandt ist und einem distalen Ende, das im Benutzungszustand vom Anwender entfernt ist, wobei zwischen dem proximalen und dem distalen Ende ein Aktor am distalen Ende angeordnet ist. Ferner dient die Freigabevorrichtung zum Lösen eines medizinischen Implantats von einer Einführvorrichtung bei welcher das Implantat zwischen einem ersten und einem zweiten Einführelement angeordnet ist, wobei durch Betätigen des Aktors durch Anlegen eines hydraulisch Drucks am distalen Ende des Körpers das Implantat durch eine Relativbewegung zwischen dem ersten und dem zweiten Einführelement freigeben wird.

Durch die erfindungsgemäße Ausgestaltung kann ein Verfahren realisiert werden, mittels dem ein Implantat bedienerfreundlich, präzise und schnell freigegeben werden kann. Auch hier umfasst der Begriff "Betätigen" das Verschieben und im Falle der Freigabe des Implantats ein Verschieben des Aktors in Richtung des proximalen Endes des Körpers.

Die Erfindung ist nachfolgend beispielhaft, anhand von in Zeichnungen dargestellten Ausführungsbeispielen, näher erläutert. Es zeigen in schematischer Darstellung:
- Fig. 1: einen Schnitt durch ein günstiges Ausführungsbeispiel einer Einführvorrichtung und einer Freigabevorrichtung;
- Fig. 2: eine Detaildarstellung der Freigabevorrichtung aus Fig. 1 und
- Fig. 3: eine Detaildarstellung einer alternativen Freigabevorrichtung.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

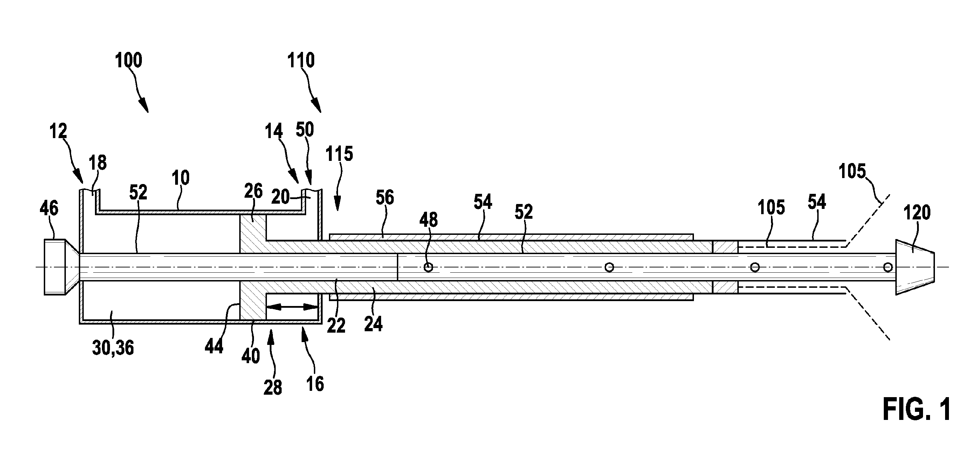

Figur 1 zeigt schematisch in einer Seitenansicht ein günstiges Ausführungsbeispiel einer Freigabevorrichtung 100 einer Einführvorrichtung 110 nach der Erfindung mit einem aufgeschnittenen Gehäuse 36, welches einen Handgriff der Einführvorrichtung 110 bildet.

Die Einführvorrichtung 110 ist beispielsweise ein Katheter mit einem Schaftbereich 50 mit zwei koaxial angeordneten Einführelementen 52, 54, z.B. einem Innenschaft (Einführelement 52) und einem diesen umgebenden Außenschaft (Einführelement 54), welcher wiederum von einer Außenhülle 56 umgeben sein kann. Die Einführvorrichtung 110 ist in Anwenderbetrieb, also während einer Herstellung der Freigabevorrichtung 100 bzw. einer Befestigung des Implantats 105 an der Freigabevorrichtung 100 oder während der Implantation mit ihrem proximalen Ende 115 einem Anwender zugewandt. Das Implantat 105 ist am distalen Ende 120 des Schaftbereichs 50 zwischen Innenschaft und Außenschaft platziert und soll am Implantationsort im tierischen oder menschlichen Körper freigegeben werden.

Die Freigabevorrichtung 100 dient zum Lösen des medizinischen Implantats 105 von der Einführvorrichtung 110. Das Implantat 105 ist an einem dem Gehäuse 36 entgegengesetzten Ende 120 des Schaftbereichs 50 beispielsweise in der Nähe einer Katheterspitze angeordnet. Das Implantat 105 ist beispielsweise um das innere Einführelement 52 gelegt und wird durch eine Relativbewegung zwischen dem ersten und dem zweiten Einführelement 52, 54 freigesetzt, wie dies teilweise in Figur 1 angedeutet ist. Hierfür ist das Implantat 105 selbstexpandierend ausgeführt.

Die Freigabevorrichtung 100 umfasst einen Körper 10 mit einem proximalen Ende 12, das im Benutzungszustand dem Anwender zugewandt ist und einem distalen Ende 14, das im Benutzungszustand vom Anwender entfernt ist. Zwischen dem proximalen und dem distalen Ende 12, 14 ist ein Aktor 16 vorgesehen. Der Aktor 16 ist hydraulisch antreibbar und dient zur Erzeugung einer gezielten Relativbewegung zwischen dem ersten und dem zweiten Einführelement 52, 54 der Einführvorrichtung 110. Zudem weist der Aktor 16 eine Kolbenstange 24 auf an deren proximalem Ende 28 ein Kolben 26 angeordnet bzw. befestigt ist. Die Kolbenstange 24 und der Kolben 26 sind einstückig mit dem äußeren Einführelement 54 ausgeführt. Jeweils sich parallel zum Einführelement 54 erstreckende Flächen 40 des Kolbens 26 bilden abdichtende Kontaktflächen mit dem Körper 10, so dass dieser einen Hydraulikzylinder 30 für den Kolben 26 bildet. Zur axialen Führung auf dem inneren Einführelement 52 weist der Aktor 16 bzw. die Kolbenstange 24 und der Kolben 26 eine Durchführung 22 auf.

Des Weiteren weist der Körper 10 am proximalen Ende 12 ein Ventil 18 und am distalen Ende 14 ein Ventil 20 auf. Beide Ventile 18, 20 sind zum Verbinden mit einer Spritze 42 als Luer Lock ausgeführt. Über jedes der Ventile 18, 20 ist mittels der Spritze 42 eine Hydraulikflüssigkeit, wie beispielsweise Wasser, zum Bewegen des Aktors 16 in den Körper 10 einbringbar. Kommt die Hydraulikflüssigkeit in Kontakt mit einer Wirkfläche 44 des Kolbens 26 bewirkt dies, dass die Kolbenstange 24 über das eine Einführelement 52, also z.B. den Innenschaft des Katheters, gleitet. Dadurch wird auch das Einführelement 54 bzw. der Außenschaft in Kraftrichtung verschoben. Eine Geschwindigkeit der Bewegung des Kolbens 26 und damit der Relativbewegung zwischen dem ersten und dem zweiten Einführelement 52, 54 der Einführvorrichtung 110 ist durch einen hydraulischen Druck p₁, p₂ vorgebbar. Zudem ist die Geschwindigkeit der Relativbewegung mittels eines Spritzenmanometers 38 überwachbar und/oder regulierbar.

Die Freigabevorrichtung 100 kann mit mindestens zwei Druckbereichen p₁, p₂ betrieben werden. Hierbei ist ein erster niederer Druckbereich p₁, p₂ für eine langsame Relativbewegung zwischen dem ersten und dem zweiten Einführelement 52, 54 vorgesehen und dient somit zum langsamen Freisetzen des Implantats 105. Ein zweiter, hoher Druckbereich p₁, p₂ hingegen ist für eine schnellere Relativbewegung vorgesehen und dient zum schnellen Freisetzen. Über den mittels der Spritze 42 bzw. der Hydraulikflüssigkeit aufgebrachten Druck p₁, p₂ auf den Aktor 16 kann die Relativbewegung schnell oder langsam erfolgen.

Die Länge jedes Körpers 10 bzw. der Bewegungsspielraum des Kolbens 26 ist zweckmä-βigerweise so bemessen, dass dieser mindestens so lang ist wie die freizugebende Länge des Implantats 105. Im Falle eines Katheters als Einführvorrichtung 110 mit einem Stent als Implantat 105 kann in der Praxis der Stent anfangs mit langsamer Geschwindigkeit bis zu einer gewissen Länge freigesetzt und somit sehr präzise positioniert werden. Danach kann der Stent vollständig mit höherer Geschwindigkeit freigesetzt werden. Das langsame Freigeben eignet sich besonders für den Beginn der Implantatsfreigabe am Implantationsort.

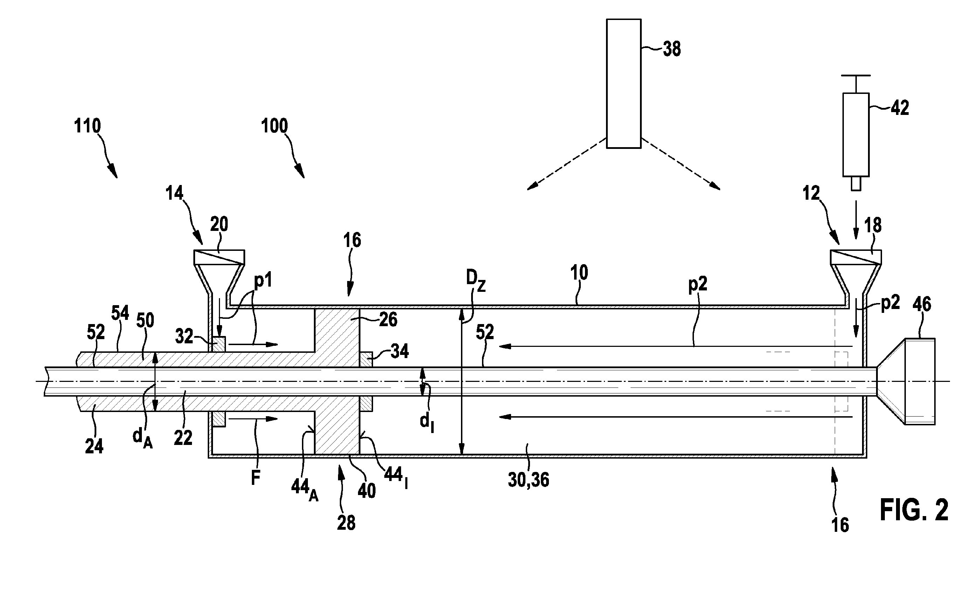

Der Körper 10 bzw. das Gehäuse 36 ist zur Überwachung der Bewegung des Kolbens 26 durchsichtig ausgeführt. Zu einer Abdichtung eines Innenraums des Körpers 10 von der Umgebung um den Körper 10 weist die Freigabevorrichtung 100 eine Dichtung 32 auf (siehe Figur 2). Die Dichtung 32 ist im Innenraum zwischen dem Körper 10 und dem Einführelement 54, also dem Außenschaft bzw. der Kolbenstange 24, in Umfangsrichtung um die Kolbenstange 24 angeordnet. Eine weitere Dichtung 34 ist zwischen dem Aktor 16 und dem Einführelement 52, also dem Innenschaft, vorgesehen und in Umfangsrichtung um diesen angeordnet. Das Lumen des Einführelements 52 (Innenschaft) kann mit einem Entlüftungsventil 46 (auch bekannt als Luer Lock) entlüftet und/oder gespült werden. Des Weiteren sind zwischen dem Außen- und dem Innenschaft mehrere Entlüftungslöcher 48 eingebracht.

Im Folgenden wird anhand der Figur 2 ein Verfahren zum Herstellen der das Implantat 105 aufeisenden Freigabevorrichtung 100 beschrieben. In einem ersten Schritt wird der Aktor 16 am distalen Ende 14 des Körpers 10 platziert. Dies kann eine Position sein, die vom Hersteller der implantatlosen Freigabevorrichtung 100 voreingestellt ist. Nachfolgend wird in einem zweiten Schritt der Aktor 16 durch Anlegen eines hydraulischen Drucks p₁ am distalen Ende 14 des Körpers 10 betätigt. Dies erfolgt durch Einspritzen der Hydraulikflüssigkeit mittels der Spritze 42 über das Ventil 20 in den Körper 10. Dies führt zu einer Platzierung des Aktors 16 am proximalen Ende 12 des Körpers 10 (gestrichelte Darstellung). Durch die dadurch verursachte Bewegen des Einführelements 54 in Richtung eines proximalen Endes 115 der Einführvorrichtung 110 kommt es zu einem Freilegen des Einführelements 52 am distalen Ende 120 der Einführvorrichtung 110. Gegebenenfalls kann der Teile des Körpers 10, der mit Hydraulikflüssigkeit befüllt wurde, vor der Befüllung über das Ventil 20 evakuiert werden. Es wäre aber auch möglich, dass das Ventil 20 einen Gassauslass aufweist, der ein Entweichen von sich im Körper 10 befindlicher Luft entgegen der Richtung des Befüllens ermöglicht. Zur leichteren Bewegung des Aktors 16 wird bevorzugt das Ventil 18 am proximalen Ende 12 für das Entweichen von Luft geöffnet.

Im nachfolgenden dritten Schritt wird das Implantat 105 am freigelegten Einführelement 52 befestigt, bevorzugt aufgecrimpt. Hierfür wird bevorzugt der Druck p₁ auf der Spritze 42 mittel Hilfe eines 4-Wege Hahns einer Hahnbank (nicht gezeigt) weggenommen. In einem vierten und abschließenden Schritt wird der Aktor 16 durch Anlegen eines hydraulischen Drucks p₂ am proximalen Ende 12 des Körpers 10 durch Einspritzen der Hydraulikflüssigkeit mittels der Spritze 42 über das Ventil 18 in den Körper 10 erneut betätigt. Nun ist der Aktor 16 wieder am distalen Ende 14 des Körpers 10 platziert. Ferner wird das Implantat 105 durch das in Richtung des distalen Endes 120 der Einführvorrichtung 110 verschobenen Einführelement 54 durch dieses bedeckt. Nun ist die Freigabevorrichtung 100 bzw. die Einführvorrichtung 110 für die Implantation bereit.

Ein Verfahren zum Betreiben der Freigabevorrichtung 100 bzw. der Einführvorrichtung 110 erfolgt nach der Einführung der Einführvorrichtung 110 in den menschlichen Körper indem der am distalen Ende 14 angeordnete Aktor 16 durch Anlegen eines hydraulischen Drucks p₁ am distalen Ende 14 des Körpers 10 ein weiteres Mal durch Einspritzen der Hydraulikflüssigkeit über das Ventil 20 betätigt wird. Dadurch verschiebt sich der Aktor 16 und der Außenschaft erneut ans proximale Ende 12 bzw. in Richtung des proximalen Endes 115 der Einführvorrichtung 110 und das Implantat 105 wird durch die Relativbewegung zwischen dem ersten und dem zweiten Einführelement 52, 54 freigeben. Hierbei wird die Geschwindigkeit, die durch die Bewegung des farbigen (z.B. schwarzen) Kolbens 26 im durchsichtigen Zylinder 30 entsteht, überwacht und mit dem Spritzenmanometer 38 reguliert. Der Zylinder 30 kann mit einem dem Fachmann bekannten lesbaren Maßstab, beispielsweise einer mL-Skala, gekennzeichnet sein. Die Freigabe im Körper kann mittels eines Röntgenverfahrens überwacht werden.

Wie oben erwähnt, kann der Druck p₁, p₂ je nach Bedarf variiert werde, beispielsweise kann für eine langsame Freigabe ein Druck von 2 bar und für eine schnellere Freigabe ein Druck von 6 bar aufgebracht werden.

Weisen die Bauteile der Freigabevorrichtung 100 die in den folgenden Tabellen exemplarisch aufgelisteten Dimensionen auf, ergibt sich gemäß Tabelle 1, dass bei einem über das Ventil 20 eingebrachten exemplarischen Druck p₁ von 6 bar auf den Kolben 26 eine Kraft F von 39,58 N aufgebracht wird, um den Kolben 26 in Richtung des proximalen Endes 12 zu bewegen. Gemäß Tabelle 2 ergibt sich, dass bei einem über das Ventil 18 eingebrachten exemplarischen Druck p₂ von 5 bar auf den Kolben 26 eine Kraft F von 37,7 N aufgebracht wird, um den Kolben 26 in Richtung des distalen Endes 14 zu bewegen. Hierbei stellen D_{Z} einen Durchmesser des Zylinders 30, D_{K} einen Durchmesser des Kolbens 26, d_{A} einen Durchmesser der Kolbenstange 24 bzw. des Außenschafts (Einführelement 54) und d_{I} einen Durchmesser des Einführelements 52 bzw. des Innenschafts dar (siehe Figur 2). Die Flächen der Kolbenstange 24, des Einführelements 52 und des Kolbens 26 berechnet sich aus κr². Im Falle von p₁ ergibt sich die Wirkfläche 44_{A} des Kolbens 26 durch Subtraktion der Fläche der Kolbenstange 24 von der Fläche des Kolbens 26. Im Falle von p₂ ergibt sich die Wirkfläche 44_{I} des Kolbens 26 durch Subtraktion der Fläche des Einführelements 52 von der Fläche des Kolbens 26.

**Tabelle 1**

| | | |
|---|---|---|
| Zylinder 30 Durchmesser D_{Z} | mm | 10,20 |
| Kolben 26 Durchmesser D_{K} | mm | 10,00 |
| Kolbenstange 24 Durchmesser d_{A} | mm | 4,00 |
| Fläche der Kolbenstange 24 | mm² | 12,57 |
| Fläche des Kolbens 26 | mm² | 78,54 |
| Wirkfläche 44_{A} | mm² | 65,97 |
| 1 bar* | N/cm² | 10,00 |
| Wasser spritzen mit 6 bar Druck p₁ | N/cm² | 60,00 |
| Kraft F auf den Kolben 26 | N | 39,58 |
| Länge des Zylinders 30 | mm | 100,00 |
| Volumen des Zylinders 30 | mm³ | 8171,28 |
| Volumen des Zylinders 30 ml** | ml | 8,17 |

| | | |
|---|---|---|
| *) 1 bar = 10 N/cm² **) 1 ml = 1 qcm= 1000 mm³ | | |

**Tabelle 2**

| | | |
|---|---|---|
| Zylinder 30 Durchmesser D_{Z} | mm | 10,20 |
| Kolben 26 Durchmesser D_{K} | mm | 10,00 |
| Einführelement 52 Durchmesser d_{I} | mm | 2,00 |
| Fläche des Einführelements 52 | mm² | 3,14 |
| Fläche des Kolbens 26 | mm² | 78,54 |
| Wirkfläche 44_{I} | mm² | 75,40 |
| 1 bar* | N/cm² | 10,00 |
| Wasser spritzen mit 5 bar Druck p₂ | N/cm² | 50,00 |
| Kraft F auf den Kolben 26 | N | 37,70 |
| Länge des Zylinders 30 | mm | 100,00 |
| Volumen des Zylinders 30 | 3 mm³ | 8171,28 |
| Volumen des Zylinders 30 ml** | ml | 8,17 |

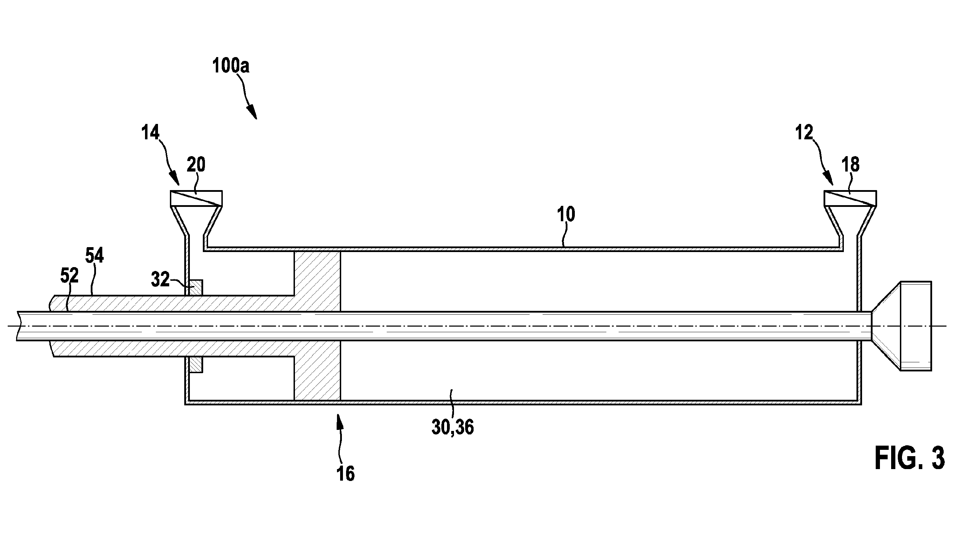

In Figur 3 ist eine alternative Freigabevorrichtung 100a gezeigt. Sie unterscheidet sich von der Ausführung der Figur 2 indem die Dichtung zwischen dem Aktor und dem Innenschaft fehlt. Dies ist möglich, da hier das Implantat 105 schon vom Hersteller der Freigabevorrichtung 100a in der Einführvorrichtung 110 befestigt wurde und somit der Montageschritt des Implantats 105 auf der Einführvorrichtung 110 für den Anwender der Freigabevorrichtung 100a entfällt.

## Patentansprüche

1. Freigabevorrichtung (100, 100a) zum Lösen eines medizinisches Implantats (105) von einer Einführvorrichtung (110), bei welcher das Implantat (105) durch eine Relativbewegung zwischen einem ersten und einem zweiten Einführelement (52, 54) freisetzbar ist, umfassend einen Körper (10) mit einem proximalen Ende (12), das im Benutzungszustand einem Anwender zugewandt ist und einem distalen Ende (14), das im Benutzungszustand vom Anwender entfernt ist, wobei zwischen dem proximalen und dem distalen Ende (12, 14) ein Aktor (16) vorgesehen ist, wobei der Aktor (16) zur Erzeugung einer gezielten Relativbewegung zwischen dem ersten und dem zweiten Einführelement (52, 54) der Einführvorrichtung (110) hydraulisch antreibbar ist.

2. Freigabevorrichtung nach Anspruch 1, wobei der Körper (10) zumindest am distalen Ende (14) ein Ventil (18, 20) aufweist und insbesondere am proximalen Ende (12) und am distalen Ende (14) je ein Ventil (18, 20) aufweist.

3. Freigabevorrichtung nach Anspruch 2, wobei über das Ventil (18, 20) eine Hydraulikflüssigkeit zum Bewegen des Aktors (16) in den Körper (10) einbringbar ist.

4. Freigabevorrichtung nach einem der vorhergehenden Ansprüche, wobei eine Geschwindigkeit der Relativbewegung zwischen dem ersten und dem zweiten Einführelement (52, 54) der Einführvorrichtung (110) durch einen hydraulischen Druck (p₁, p₂) vorgebbar ist.

5. Freigabevorrichtung nach Anspruch 4, wobei mindestens zwei Druckbereiche (p₁, p₂) vorgesehen sind, wobei ein erster niederer Druckbereich (p₁, p₂) für eine langsame Relativbewegung und ein zweiter, hoher Druckbereich (p₁, p₂) für eine schnellere Relativbewegung vorgesehen ist.

6. Freigabevorrichtung nach einem der vorhergehenden Ansprüche, wobei der Aktor (16) eine Durchführung (22) für das innere Einführelement (52) aufweist.

7. Freigabevorrichtung nach einem der vorhergehenden Ansprüche, wobei der Aktor (16) eine Kolbenstange (24) und einen Kolben (26) aufweist, wobei der Kolben (26) an einem proximalen Ende (28) der Kolbenstange (24) angeordnet ist.

8. Freigabevorrichtung nach Anspruch 7, wobei der Körper (10) einen Hydraulikzylinder (30) für den Kolben (26) aufweist.

9. Freigabevorrichtung nach Anspruch 7, wobei die Kolbenstange (24) und/oder der Kolben (26) einstückig mit dem äußeren Einführelement (54) ausgeführt sind.

10. Freigabevorrichtung nach einem der vorhergehenden Ansprüche, wobei eine Dichtung (32, 34) vorgesehen ist, die zwischen zumindest einem Einführelement (52, 54) und dem Körper (10) und/oder dem Aktor (16) angeordnet ist.

11. Freigabevorrichtung nach einem der vorhergehenden Ansprüche, wobei der Körper (10) als ein Gehäuse (36) ausgeführt ist, das in wenigstens einem Bereich durchsichtig ausgeführt ist.

12. Freigabevorrichtung nach einem der vorhergehenden Ansprüche, wobei eine Geschwindigkeit der Relativbewegung optisch überwachbar ist oder mittels eines Spritzenmanometers (38) überwachbar und/oder regulierbar ist.

13. Einführvorrichtung (110) zum Einführen eines medizinischen Implantats (105), welches durch eine Relativbewegung zwischen einem ersten und einem zweiten Einführelement (52, 54) freisetzbar ist, umfassend eine Freigabevorrichtung (100, 100a) zum Lösen des medizinischen Implantats (105), insbesondere nach einem der vorhergehenden Ansprüche, umfassend einen Körper (10) mit einem proximalen Ende (12), das im Benutzungszustand einem Anwender zugewandt ist und einem distalen Ende (14), das im Benutzungszustand vom Anwender entfernt ist, wobei zwischen dem proximalen und dem distalen Ende (12, 14) ein Aktor (16) vorgesehen ist, wobei der Aktor (16) zur Erzeugung einer gezielten Relativbewegung zwischen dem ersten und dem zweiten Einführelement (52, 54) der Einführvorrichtung (110) hydraulisch antreibbar ist.

14. Verfahren zum Herstellen einer Implantat aufweisenden Freigabevorrichtung (100, 100a), insbesondere nach einem der Ansprüche 1 bis 12, umfassend einen Körper (10) mit einem proximalen Ende (12), das im Benutzungszustand einem Anwender zugewandt ist und einem distalen Ende (14), das im Benutzungszustand vom Anwender entfernt ist, wobei zwischen dem proximalen und dem distalen Ende (12, 14) ein Aktor (16) vorgesehen ist und zum Lösen eines medizinischen Implantats (105) von einer Einführvorrichtung (110), insbesondere nach Anspruch 14, bei welcher das Implantat (105) durch eine Relativbewegung zwischen einem ersten und einem zweiten Einführelement (52, 54) freisetzbar ist, aufweisend zumindest die folgenden Schritte:
- Platzieren des Aktors (16) am distalen Ende (14) des Körpers (10);
- Betätigen des Aktors (16) durch Anlegen eines hydraulischen Drucks (p₁) am distalen Ende (14) des Körpers (10) und damit Freilegen des Einführelements (52) an einem distalen Ende (120) der Einführvorrichtung (110) durch Bewegen des Einführelements (54) in Richtung eines proximalen Endes (115) der Einführvorrichtung (110);
- Befestigen des Implantats (105) am freigelegten Einführelement (52);
- Betätigen des Aktors (16) durch Anlegen eines hydraulischen Drucks (p₂) am proximalen Ende (12) des Körpers (10) und damit Bedecken des Implantats (105) durch das in Richtung des distalen Endes (120) der Einführvorrichtung (110) verschobenen Einführelement (54) durch dieses.

15. Verfahren zum Betreiben einer Freigabevorrichtung (100, 100a), insbesondere nach einem der Ansprüche 1 bis 12, umfassend einen Körper (10) mit einem proximalen Ende (12), das im Benutzungszustand einem Anwender zugewandt ist und einem distalen Ende (14), das im Benutzungszustand vom Anwender entfernt ist, wobei zwischen dem proximalen und dem distalen Ende (12, 14) ein Aktor (16) am distalen Ende (14) angeordnet ist und zum Lösen eines medizinischen Implantats (105) von einer Einführvorrichtung (110), insbesondere nach Anspruch 13, bei welcher das Implantat (105) zwischen einem ersten und einem zweiten Einführelement (52, 54) angeordnet ist, wobei durch Betätigen des Aktors (16) durch Anlegen eines hydraulisch Drucks (p₁) am distalen Ende (14) des Körpers (10) das Implantat (105) durch eine Relativbewegung zwischen dem ersten und dem zweiten Einführelement (52, 54) freigeben wird.
